# EUROPÄISCHE PATENTANMELDUNG

(11) **EP 4 566 510 A1**
(43) Veröffentlichungstag der Anmeldung: **11.06.2025**
(21) Anmeldenummer: 24217447.2
(22) Anmeldetag: 04.12.2024
(51) Int. Cl.: A61B 1/00

(54) **CHIRURGISCHES INSTRUMENT UND CHIRURGISCHES SYSTEM**

(30) Priorität: 08.12.2023 DE 102023134458
(71) Anmelder: Karl Storz SE & Co. KG, 78532 Tuttlingen (DE)
(72) Erfinder: FORSTER, Jonas, 78532 Tuttlingen (DE); KLEIN, Kirsten, 78532 Tuttlingen (DE); KROTZ, Tobias, 78532 Tuttlingen (DE); FRICK, Andreas, 78532 Tuttlingen (DE); KASPARIK, Günther, 78532 Tuttlingen (DE)

(57) **Zusammenfassung**

Ein chirurgisches Instrument (12) weist einen Schaft (14), der sich zwischen einem proximalen Ende (42) und einem distalen Ende (40) erstreckt und eine Längsachse (18) definiert, ein Handhabungsgehäuse (16), das an seinem dem Schaft (14) zugewandten ersten Ende (50) an das proximale Ende (42) des Schafts (14) ankoppelt und an seinem vom Schaft (14) abgewandten zweiten Ende (52) einen Leitungsanschluss (34) aufweist, und einen Zusatzgriff auf, wobei das Handhabungsgehäuse (16) zwischen dem ersten Ende (50) und dem zweiten Ende (52) zumindest abschnittsweise gekrümmt ist, so dass der Leitungsanschluss (34) eine gegenüber der Längsachse (18) geneigte Ausrichtung aufweist.

## Beschreibung

Die vorliegende Offenbarung bezieht sich auf ein chirurgisches Instrument, insbesondere ein endoskopisches Beobachtungsinstrument, mit einem Schaft, der sich zwischen einem proximalen Ende und einem distalen Ende erstreckt und eine Längsachse definiert, mit einem Handhabungsgehäuse, das an seinem dem Schaft zugewandten ersten Ende an das proximale Ende des Schafts ankoppelt und an seinem vom Schaft abgewandten zweiten Ende einen Leitungsanschluss aufweist.

Die vorliegende Offenbarung bezieht sich insbesondere auf sogenannte Chip-on-the-Tip-Instrumente, beispielsweise Chip-on-the-Tip-Endoskope. Bei derartigen Instrumenten sitzt ein Bildaufnehmer im distalen Endbereich des Schafts. Insbesondere gibt es keine optische Bildübertragung entlang eines optischen Beobachtungspfades (beispielsweise mittels Stablinsen oder dergleichen) hin zum proximalen Ende des Schafts und gegebenenfalls darüber hinaus hin zu einem Okular.

Ein Endoskop mit einem Bildaufnehmer beim distalen Ende des Schafts ist beispielsweise aus der DE 10 2019 003 842 A1 bekannt. Der Begriff Chip-on-the-Tip bzw. die Anordnung des Bildaufnehmers beim distalen Ende des Schafts schließt nicht aus, dass dem Bildaufnehmer (Bildsensor) distalseitig eine Optik vorgelagert ist, beispielsweise zumindest ein Objektivlinsensystem.

Chirurgische Instrumente im Rahmen der vorliegenden Offenbarung dienen zur Beobachtung (und gegebenenfalls Behandlung) des menschlichen und tierischen Körpers, insbesondere im Körperinneren.

Beispielsweise ist ein Resektoskop ein Instrument, das zur Beobachtung und zur Behandlung des menschlichen und tierischen Körpers dient. Ein Resektoskop umfasst einen Beobachtungsabschnitt (beispielsweise Endoskop) und einen Behandlungsabschnitt (üblicherweise mit hochfrequentem Wechselstrom beaufschlagte Elektrode(n)) zum Abtragen von Gewebe. Die vorliegende Offenbarung bezieht sich zumindest in beispielhaften Ausgestaltungen auf Resektoskope, beispielsweise für die transurethrale Resektion, ist jedoch nicht auf solche Instrumente und Anwendungen eingeschränkt.

Weitere beispielhafte Anwendungen umfassen die perkutane Nephrolitholapaxie (PCNL) und weitere minimalinvasive Eingriffe.

Aus der EP 0 501 088 A1 ist ein chirurgisches Instrument in Form eines Resektoskopes bekannt, wobei das Instrument ein Endoskop mit einem sich entlang einer Längsachse erstreckenden Schaft und mit einem rein optischen Beobachtungspfad zwischen einem distalen Ende des Instruments und einem Okular beim proximalen Ende des Schafts aufweist. Zur Erzeugung eines Videosignals kann eine Kamera mit einem Kameraobjektiv an das Okular des Endoskops angekoppelt werden. Die Kamera und das Endoskop sind relativ zueinander rotierbar, so dass das Kameraobjektiv bei einer Verdrehung des Endoskops seine Lage durch Auspendeln beibehalten kann.

Die DE 10 2004 046 038 A1 beschreibt in Zusammenhang mit einer Simulationsanwendung ein Resektoskop mit einem Okular, an das eine Pendelkamera ankoppelbar ist, die ein Kameragehäuse mit einem Handgriff aufweist. Derartige Handgriffe werden auch als Pistolengriff bezeichnet. Ein Benutzer kann das Resektoskop über den Griff des Kameragehäuses halten und führen, wobei das Instrument relativ zum Kameragehäuse rotierbar ist. Die Übertragung von Bildern zwischen dem Okular und der Kamera erfolgt rein optisch, so dass bei der Relativrotation keine Rücksicht auf Kabel und dergleichen genommen werden muss.

Aus der US 5 785 644 A, der US 2006/0287575 A1, der DE 10 2013 103 905 A1, der US2017/0209025 A1, der US 4,384,570 A, der DE 44 08 393 A1, der DE 10 2004 030 030 A1, der DE 10 2010 049 759 A1 und der DE 10 2017 219 621 A1 sind ebenfalls entsprechende chirurgische Instrumente bekannt.

Es hat sich gezeigt, dass für bestimmte chirurgische Anwendungen das Halten und Führen des Instruments über einen etwa als Pistolengriff gestalteten Handgriff vorteilhaft ist. Insbesondere kann ein solcher Griff als Basis oder Referenz dienen, wenn das Instrument rotiert wird. Bei einer Rotation des Instruments wird der Bildaufnehmer (Bildsensor) einer Pendelkamera über den Griff mit festgehalten. Damit ist der Horizont des erfassten Bild definiert, auch wenn eine Relativrotation zwischen dem Instrument und dem Bildaufnehmer stattfindet.

Instrumente, bei denen der Bildaufnehmer beim distalen Ende des Schafts angeordnet ist, machen Kameraköpfe (Pendelkameras) obsolet, weil das Videosignal bereits durch den Bildaufnehmer im distalen Endbereich des Schafts erzeugt wird. Ferner weisen solche Instrumente beim proximalen Ende des Schafts keinen Endoskopkopf mit klassischem Okular auf, durch den sich der optische Beobachtungspfad erstreckt. Stattdessen ist beispielsweise beim proximalen Ende des Schafts ein Handhabungsgehäuse verbaut, das elektronische Komponenten beherbergt und Betätigungselemente zur Steuerung des Instruments bereitstellt.

Ein solches Handhabungsgehäuse rotiert jedoch als Bestandteil des Instruments gemeinsam mit dem Schaft, wenn das Instrument um die Längsachse rotiert wird. Bei einer solchen Rotation fehlt einem Bediener mitunter die Basis oder Referenz für die händische Führung des Instruments. Pendelkameras mit geeigneten Griffen, die bei konventionellen (rein optischen) Endoskop eine solche Referenz bereitstellen könnten, können bei Instrumenten mit distal angeordneten Bildaufnehmer nicht verwendet werden.

Vor diesem Hintergrund liegt der vorliegenden Offenbarung die Aufgabe zugrunde, ein chirurgisches Instrument, insbesondere ein endoskopisches Beobachtungsinstrument zur Bereitstellung von Videosignalen aus dem Körperinneren, anzugeben, das einerseits kompakt gestaltet ist und andererseits einem Bediener die gewohnte Handhabung ermöglicht. Es soll ein chirurgisches Instrument zur Verfügung gestellt werden, dass eine besonders nutzungsfreundliche Handhabung ermöglicht. Das Instrument soll zumindest für bestimmte Anwendungen direkt an seinem Handhabungsgehäuse und für andere Anwendungen über einen Zusatzgriff, insbesondere ein Griffstück, sicher gehalten und geführt werden können. Das Instrument soll vielfältig einsetzbar sein und im Rahmen verschiedener chirurgischer Prozeduren verwendbar sein. Das Instrument soll für kombinierte chirurgische Eingriffe geeignet sein, bei denen mit nur einem Gerät sowohl eine Beobachtung als auch eine Behandlung von Gewebe erfolgt. Zumindest in beispielhaften Ausgestaltungen soll das Instrument für den Bediener ähnlich wie ein klassisches Endoskop mit Stablinsenoptik, Okular und aufgesetztem Pendelkamerakopf handhabbar und bedienbar sein. Zudem soll ein entsprechendes chirurgisches System mit einem solchen Instrument beschrieben werden.

Gemäß einem ersten Aspekt bezieht sich die vorliegende Offenbarung auf ein chirurgisches Instrument, insbesondere ein endoskopisches Beobachtungsinstrument, mit einem Schaft, der sich zwischen einem proximalen Ende und einem distalen Ende erstreckt und eine Längsachse definiert, mit einem Handhabungsgehäuse, das an seinem dem Schaft zugewandten ersten Ende an das proximale Ende des Schafts ankoppelt und an seinem vom Schaft abgewandten zweiten Ende einen Leitungsanschluss aufweist, und mit einem Zusatzgriff, wobei das Handhabungsgehäuse zwischen dem ersten Ende und dem zweiten Ende zumindest abschnittsweise gekrümmt ist, so dass der Leitungsanschluss eine gegenüber der Längsachse geneigte Ausrichtung aufweist..

Die Aufgabe der Offenbarung wird auf diese Weise gelöst.

Ein offenbarungsgemäß gestaltetes Instrument erlaubt einen sicheren Griff und eine sichere Führung des Instruments. Auf diese Weise kann ein Leitungsanschluss am von dem Schaft abgewandten Ende des Handhabungsgehäuses von der Längsachse beabstandet sein und gegebenenfalls schräg dazu orientiert sein. Ähnliches kann sich für die Orientierung eines dort angeschlossenen Anschlusskabels ergeben. Eine solche Gestaltung mit einem zumindest leicht gekrümmten Gehäuse mit entsprechender Orientierung des Anschlusskabels hat sich für bestimmte Anwendungen als vorteilhaft erwiesen. Es kann ein Instrument bereitgestellt werden, bei welchem ein an dem Leitungsanschluss angeschlossenes Anschlusskabel besonders nutzungsfreundlich angeordnet sein kann. Das Anschlusskabel lässt sich vorteilhaft so positionieren, dass es für den Nutzer kaum wahrnehmbar ist und nicht im Weg ist.

Der Zusatzgriff kann ein Griffstück aufweisen oder als ein solches ausgebildet sein. Der Zusatzgriff, insbesondere das Griffstück, kann einstückig mit dem Handhabungsgehäuse ausgebildet sein. Alternativ kann der Zusatzgriff, insbesondere das Griffstück, auch separat zu dem Handhabungsgehäuse ausgebildet sein. Der Zusatzgriff, insbesondere das Griffstück, kann drehfest mit dem Handhabungsgehäuse verbunden sein. Gemäß einer Weiterbildung der Erfindung kann das Instrument eine beim Handhabungsgehäuse ausgebildete Aufnahme für den Zusatzgriff, insbesondere das Griffstück, aufweisen. Das Griffstück dient dem Halten des Instruments und weist insbesondere keine elektrischen oder elektronischen Komponenten oder Anschlüsse auf.

Der Zusatzgriff, insbesondere das Griffstück, und das Handhabungsgehäuse können alternativ im montierten Zustand des Zusatzgriffs, insbesondere des Griffstücks, um eine Schwenkachse verschwenkbar sein, die im Wesentlichen parallel zur Längsachse des Schafts orientiert ist. Ein derart gestaltetes Instrument erlaubt die einfache Ankopplung eines Griffstücks für einen sicheren Griff und eine sichere Führung des Instruments. Vorteilhaft kann eine Relativrotation des Instruments gegenüber einem beispielsweise als Pistolengriff gestalteten Griffteil, insbesondere dem Griffstück, realisiert werden. Auf diese Weise kann ein an konventionelle Instrumente mit Pendelkamera gewöhnter Anwender ein offenbarungsgemäßes Instrument in der ihm/ihr geläufigen Weise einsetzen. Dies umfasst insbesondere auch eine Rotation des Instruments (zumindest der den Schaft und das Handhabungsgehäuse umfassende Teil davon) relativ zum Zusatzgriff, insbesondere zum Griffstück. Die Rotation (Schwenkbewegung) des Instruments dient beispielsweise bei Schrägblick-Instrumenten zur Anpassung des Sichtfeldes. Üblicherweise erfolgt die Schwenkbewegung, indem das Instrument aktiv verschwenkt/rotiert wird, wenn der Zusatzgriff, insbesondere das Griffstück, festgehalten wird. Wenn also der Bediener den Zusatzgriff, insbesondere das Griffstück, mit einer Hand hält und hinreichend fixiert, kann das Instrument mit der anderen Hand um die Schwenkachse geschwenkt werden.

Ein offenbarungsgemäß gestaltetes Instrument weist als integralen Bestandteil das Handhabungsgehäuse auf. Mit anderen Worten ist das Instrument ohne Handhabungsgehäuse (bzw. darin enthaltene Komponenten) nicht betriebsfähig. Das Handhabungsgehäuse dient in manchen Ausführungsformen als Kameragehäuse. Wenn es nicht als Kamera dient, ist die "Kamera" (Bildaufnehmer bzw. Bildsensor) stattdessen beispielsweise in einem distalen Endbereich des Schafts angeordnet. Jedoch beherbergt das Handhabungsgehäuse stets elektronische Komponenten, z.B. Bedienelemente (Knöpfe, Taster und dergleichen), Kabelanschlüsse (Informationsübertragung, Energieübertragung und dergleichen), elektronische Komponenten zur Signalverarbeitung wie FPGAs oder Signalwandler, Halbleiteremitter für eine Beleuchtung und ähnliches.

Das Instrument kann ausdrücklich auch ohne den Zusatzgriff, insbesondere das (insoweit optionale) Griffstück, betrieben werden. Der Zusatzgriff, insbesondere das Griffstück, kann auch als Aufsatz-Griffstück gestaltet und bezeichnet werden. Der Zusatzgriff, insbesondere das Griffstück, kann auch als Handstück oder Pistolengriff bezeichnet werden. Der Zusatzgriff, insbesondere das Griffstück, ist beispielsweise um einen stumpfen Winkel (beispielsweise 60° bis 105°) gegenüber der Längsachse geneigt, wobei der Winkel nach primär nach distal geöffnet ist. Der Zusatzgriff, insbesondere das Griffstück, ist beispielsweise bezüglich des Handhabungsgehäuses einsteckbar oder aufsteckbar gestaltet. Vorzugsweise ist der Zusatzgriff, insbesondere das Griffstück, abnehmbar. Somit kann individuell entscheiden werden, ob das Instrument mit oder ohne Zusatzgriff, insbesondere Griffstück, genutzt wird.

Üblicherweise wird eine besonders kompakte und miniaturisierte Gestaltung des Instruments angestrebt. Dies betrifft beispielsweise den Durchmesser des Schafts, aber auch die Abmessungen des Handhabungsgehäuses. Beispielhaft kann das Handhabungsgehäuse derart gestaltet sein, dass das Instrument insgesamt ähnlich einem Stylus (vergleichbar einem dicken Stift) gehalten und bewegt werden kann. Es gibt jedoch auch Anwendungen, bei denen zumindest einige Nutzer das Instrument in gewohnter Weise über einen Griff (Pistolengriff) halten wollen, wobei auch der Rotationsfreiheitsgrad zwischen dem Instrument und dem Griff beibehalten werden soll. Für derartige Anwendungen steht nun mit dem Zusatzgriff, insbesondere dem Griffstück, ein Zusatzbauteil zur Verfügung, das den Anwendungsbereich des Instruments vergrößert.

Darunter, dass das Handhabungsgehäuse zwischen dem ersten Ende und dem zweiten Ende zumindest abschnittsweise gekrümmt ist, kann verstanden werden, dass das Handhabungsgehäuse eine zumindest teilweise gekrümmte, beispielsweise gebogene, Form aufweist. Insbesondere kann ein zumindest abschnittsweise gekrümmtes Handhabungsgehäuse nicht allein durch eine gekrümmte Wand des Gehäuses realisiert sein. Mit anderen Worten kann eine Haupterstreckungslinie des Handhabungsgehäuses zwischen dem ersten Ende und dem zweiten Ende zumindest abschnittsweise gekrümmt sein. Die Haupterstreckungslinie kann auch als Mittellinie durch das Handhabungsgehäuse verstanden werden, welche sich von dem ersten Ende bis zu dem zweiten Ende erstrecken kann. Die Haupterstreckungslinie kann ferner auch als Haupterstreckungsrichtung verstanden werden. Die Haupterstreckungsrichtung kann beispielsweise durch die jeweiligen geometrischen Schwerpunkte der Querschnitte des Handhabungsgehäuses entlang der Erstreckung des Handhabungsgehäuses definiert sein, insbesondere der Erstreckung von dem ersten Ende bis zu dem zweiten Ende des Handhabungsgehäuses.

Beispielsweise beträgt eine Neigung, insbesondere der geneigten Ausrichtung, im Bereich des Leitungsanschlusses 15° bis 45° relativ zur Längsachse. Ein Winkel der Neigung kann nach proximal geöffnet sein, insbesondere bezüglich einer Längserstreckung des Instruments.

Diese Gestaltung mit gekrümmtem Handhabungsgehäuse muss jedoch bei der Gestaltung der Schnittstelle zwischen dem Handhabungsgehäuse und dem Griffteil, insbesondere dem Griffstück, berücksichtigt werden. Die Orientierung der Rotationsachse ist durch die Längsachse des Schafts des Instruments vorgegeben.

Das Instrument kann einen Bildaufnehmer aufweisen, der beim distalen Ende des Schafts angeordnet ist. Im Rahmen der vorliegenden Offenbarung handelt es sich bei einem Bildaufnehmer (Bildsensor) beim distalen Ende des Schafts um einen Bildaufnehmer, der in einem distalen Endbereich des Schafts angeordnet ist. Mit anderen Worten muss der Bildaufnehmer nicht die distale Spitze des Schafts bilden. Stattdessen kann distal vor dem Bildaufnehmer eine Objektivoptik und/oder zumindest eine transparente Scheibe (Schutzglas oder dergleichen) angeordnet sein. Das Instrument ist zumindest beispielhaft als sogenanntes Chip-on-the-Tip-Endoskop gestaltet. Das Instrument kann als monoskopisches Instrument mit einem einzigen Beobachtungskanal oder als - zur Realisierung eines räumlichen Seeeindrucks - stereoskopisches Instrument mit zwei Beobachtungskanälen gestaltet sein. Das Endoskop kann als ein starres Endoskop ausgebildet sein.

Das Instrument wird insbesondere für minimalinvasive Prozeduren genutzt, umfassend die Beobachtung des Körperinneren und gegebenenfalls die Behandlung von Gewebe und dergleichen im Körperinneren. Insbesondere bei Instrumenten mit geneigter Blickrichtung erlaubt die Schwenkbewegung des Instruments relativ zum Zusatzgriff, insbesondere zum Griffstück, ein Wandern des Sichtfeldes, so dass ein größerer Bereich observierbar ist.

Der Leitungsanschluss beim zweiten (proximalen) Ende des Handhabungsgehäuses erlaubt die Ankopplung einer Versorgungsleitung oder dergleichen. Dies kann zu Zwecken der Energieversorgung und/oder des Informationsaustausches erfolgen. Hinsichtlich der Beleuchtung ist es zum einen vorstellbar, Lichtquellen (üblicherweise LED-Lichtquellen) innerhalb des Instruments zu verbauen. Es ist zum anderen auch vorstellbar, außerhalb des Instruments verbaute Lichtquellen zu nutzen und diese entsprechend über Lichtleiter an das Instrument anzukoppeln.

Im Rahmen der vorliegenden Offenbarung handelt es sich bei einem proximalen Abschnitt oder Bereich um einen Abschnitt oder Bereich, der näher beim Beobachter/Anwender und weiter weg vom Sichtfeld/Patienten angeordnet ist als ein distaler Abschnitt oder Bereich. Gleichermaßen handelt es sich bei einem distalen Abschnitt oder Bereich um einen Abschnitt oder Bereich, der näher beim Sichtfeld/Patienten und weiter weg vom Beobachter/Anwender angeordnet ist als ein proximaler Abschnitt oder Bereich. Demgemäß kann distal auch als patientennah, dem Patienten zugewandt und/oder beobachterfern beschrieben werden. Proximal kann auch als patientenfern, dem Patienten abgewandt und/oder beobachternah beschrieben werden. Bei der Anwendung als endoskopisches Instrument ist üblicherweise das distale Ende des Schafts in den Körper eingeführt um dort Beobachtungen vornehmen zu können. Zumindest das proximale Ende des Instruments ragt aus dem Körper heraus, weil dort die Handhabung und Steuerung durch den Bediener erfolgt.

Dies schließt jedoch nicht aus, dass Instrumente im Rahmen der vorliegenden Offenbarung grundsätzlich auch als exoskopische Instrumente gestaltet sein können, die zur Beobachtung des Körpers von außerhalb des Körpers geeignet sind.

Bei dem Bildaufnehmer handelt es sich um einen Bildsensor, beispielsweise umfassend ein CCD-Sensorarray, CMOS-Sensorarray oder dergleichen.

Die Schwenkachse kann zumindest im Wesentlichen parallel zur Längsachse des Schafts verlaufen. In beispielhaften Ausgestaltungen ist die Längsachse parallel zur Längsachse des Schafts. Auf diese Weise kann das Instrument definiert rotiert werden, ohne dass sich an der Parallelität zwischen Schwenkachse und Längsachse etwas ändert.

Das Handhabungsgehäuse kann auch als Kopfstück zur Handhabung und Steuerung des Instruments bezeichnet werden. In einer beispielhaften Ausgestaltung gleich die Gestaltung des Handhabungsgehäuses der einer halben Banane, und zwar insbesondere der Hälfte mit dem Stängel, wobei der Stängel dem Leitungsanschluss beim zweiten Ende gleicht und die gegenüberliegende Fläche (Schnittfläche bei halber Banane) in etwa dem ersten Ende entspricht, dort aber ggfs. großzügig verrundet ist. Anders formuliert ist das Handhabungsgehäuse beispielsweise als längliches, halb geschnittenes Ellipsoid mit Krümmung entlang der Längserstreckung gestaltet.

Gemäß einer beispielhaften Ausgestaltung weist das Handhabungsgehäuse zwischen dem ersten Ende und dem zweiten Ende eine gekrümmte Haupterstreckungsrichtung auf, so dass der Leitungsanschluss von der Längsachse versetzt ist. Beispielsweise weist das Handhabungsgehäuse ferner insbesondere einen sich entlang der Haupterstreckungsrichtung hin zum zweiten Ende verjüngenden Querschnitt auf, wobei das Maximum der Querschnittsfläche näher beim ersten Ende als beim zweiten Ende liegt. Aus ergonomischen Gründen ist das Handhabungsgehäuse gerade nicht als geradlinige (zylindrische) Verlängerung des Schafts mit konstantem Querschnitt gestaltet. Stattdessen ist das Handhabungsgehäuse zumindest in beispielhaften Ausgestaltungen ähnlich einer halben Banane gekrümmt, um eine günstige Ergonomie bereitzustellen. Dies ist bei der Gestaltung der Schnittstelle für den Zusatzgriff, insbesondere das Griffstück, zu berücksichtigen. Gemäß einer weiteren beispielhaften Ausgestaltung weist das Handhabungsgehäuse entlang seiner Längserstreckung zumindest abschnittsweise einen nicht rotationssymmetrischen Querschnitt auf.

Gemäß einer weiteren beispielhaften Ausgestaltung weist das Handhabungsgehäuse eine Betätigungseinheit zur Steuerung des Instruments auf. Die Betätigungseinheit weist zumindest ein Betätigungselement auf und kann insbesondere mehrere Betätigungselemente aufweisen. Folglich kann die Steuerung des Instruments zumindest teilweise direkt am Instrument erfolgen. Insbesondere handelt es sich bei dem zumindest einen Betätigungselement um einen Schalter, einen Taster, ein Touchscreen, einen Steuerknüppel oder dergleichen zur Steuerung elektrischer oder elektronischer Funktionen. Es handelt sich zumindest in beispielhaften Ausgestaltungen gerade nicht um rein mechanische Steuerelemente. Die Betätigungseinheit kann bezüglich der gekrümmten Haupterstreckungsrichtung des Handhabungsgehäuses zumindest teilweise auf einer Innenseite des Handhabungsgehäuses angeordnet sein. Unter der Innenseite des Handhabungsgehäuses bezüglich der gekrümmten Haupterstreckungsrichtung kann eine Innenseite, also insbesondere eine konkave Seite, der Krümmung des Handhabungsgehäuses verstanden werden. Vorzugsweise befinden sich mehr als die Hälfte, die meisten oder alle Betätigungselemente auf der Innenseite des gekrümmten Handhabungsgehäuses.

Gemäß einer weiteren beispielhaften Ausgestaltung ist eine Blickrichtung des Instruments gegenüber einer Längsrichtung, insbesondere der Längsachse, des Schafts geneigt, wobei der Neigungswinkel zwischen der Blickrichtung und der Längsrichtung, insbesondere der Längsachse, des Schafts insbesondere 15° bis 45° beträgt. Dies schließt jedoch kleinere und größere Winkel (etwa Bereich von 0° bis 60°) nicht aus. Ein solches Instrument mit einem von 0° abweichenden Blickwinkel kann auch als Schrägblick-Instrument bezeichnet werden. Üblicherweise weisen endoskopische Instrumente einen festen Blickwinkel auf. Es sind jedoch auch Instrumente mit verstellbarem Blickwinkel bekannt. Instrumente mit geneigter Blickrichtung haben den Vorteil, dass durch eine Rotation des Instruments um die Längsachse das Sichtfeld wandert, so dass insgesamt ein größerer Bereich beobachtet werden kann. Der Neigungswinkel zwischen der Blickrichtung und der Längsachse des Schafts kann in Richtung distal geöffnet sein.

Gemäß einer Weiterbildung der Erfindung kann vorgesehen sein, dass die Blickrichtung des Instruments und die gekrümmte Haupterstreckungsrichtung des Handhabungsgehäuses bezüglich der Längsachse voneinander abgewandt orientiert sind. Die Blickrichtung und die Haupterstreckungsrichtung können in einer gemeinsamen Ebene verlaufen, die die Längsachse einschließen kann. Alternativ können die Blickrichtung und die Längsachse einerseits und die Haupterstreckungsrichtung und die Längsachse andererseits in voneinander verschiedenen Ebenen verlaufen. Die Blickrichtung und die Haupterstreckungsrichtung können zumindest abschnittsweise zueinander parallel, insbesondere antiparallel, oder auch zueinander abgewinkelt verlaufen. Beispielsweise kann die Blickrichtung bezüglich der Längsachse nach "unten" geneigt sein und die gekrümmte Haupterstreckungsrichtung des Handhabungsgehäuses nach "oben" geneigt sein. Der Leitungsanschluss und die Blickrichtung können bezüglich der Längsachse auf voneinander abgewandten Seiten bezüglich der Längsachse angeordnet sein.

Gemäß einer weiteren beispielhaften Ausgestaltung sind das Handhabungsgehäuse und der Schaft drehfest miteinander verbunden. Auf diese Weise ist gewährleistet, dass bei einer Rotation des Instruments nicht nur der Schaft, sondern auch das Handhabungsgehäuse rotiert wird. Bei dieser Rotation wird bei der Anordnung mit dem Bildaufnehmer im distalen Endbereich des Schafts zwangsläufig auch die "Kamera" rotiert.

Es kann vorgesehen sein, dass die Aufnahme eine Anordnungsposition für den Zusatzgriff, insbesondere das Griffstück, definiert. Die Anordnungsposition liegt bezüglich der gekrümmten Haupterstreckungsrichtung des Handhabungsgehäuses auf einer von der Innenseite des Handhabungsgehäuses abgewandten Seite des Handhabungsgehäuses. Durch eine derartige Ausgestaltung des Handhabungsgehäuses in Kombination mit der Anordnungsposition des Zusatzgriffs lässt sich besonders großzügiger Raum für eine komfortable Bedienung schaffen. Die Anordnungsposition kann sich bezüglich der Längsachse beispielsweise auf einer Seite mit der Blickrichtung befinden.

Gemäß einer weiteren beispielhaften Ausgestaltung ist die Schwenkachse durch eine Schwenkführung definiert, wobei die Schwenkführung ein männliches Führungsteil und ein weibliches Führungsteil aufweist, die relativ zueinander verschwenkbar sind. Die Schwenkführung dient sowohl zur Aufnahme des Zusatzgriffs, insbesondere des Griffstücks, als auch zur Bereitstellung der Führung für die Relativbewegung zwischen dem Instrument (Handhabungsgehäuse) und dem Zusatzgriff, insbesondere dem Griffstück.

Gemäß einer weiteren beispielhaften Ausgestaltung ist die Schwenkführung unmittelbar zwischen dem Handhabungsgehäuse und dem Zusatzgriff, insbesondere dem Griffstück, ausgebildet. Somit kann der Zusatzgriff, insbesondere das Griffstück, direkt an das Handhabungsgehäuse angekoppelt werden, auf diese Weise wird automatisch die Schwenkführung zwischen dem Handhabungsgehäuse und dem Zusatzgriff, insbesondere dem Griffstück, bereitgestellt.

Gemäß einer weiteren beispielhaften Ausgestaltung ist die Schwenkführung mittelbar zwischen dem Handhabungsgehäuse und dem Zusatzgriff, insbesondere dem Griffstück, ausgebildet, wobei die Schwenkführung insbesondere zwischen dem Zusatzgriff, insbesondere dem Griffstück, und einem relativ zum Zusatzgriff, insbesondere Griffstück, verschwenkbaren Befestigungsstück ausgebildet ist, das mit dem Handhabungsgehäuse koppelbar ist. Auf diese Weise kann sich eine mittelbare Kopplung zwischen dem Zusatzgriff, insbesondere dem Griffstück, und dem Handhabungsgehäuse ergeben.

Der Zusatzgriff, insbesondere das Griffstück, kann über das Befestigungsstück am Handhabungsgehäuse befestigt werden, die Schwenkbewegung kann zwischen dem Zusatzgriff, insbesondere dem Griffstück, und den miteinander gefügten Komponenten Befestigungsstück und Handhabungsgehäuse erfolgen.

Gemäß einer weiteren beispielhaften Ausgestaltung ist das Befestigungsstück drehfest mit dem Handhabungsgehäuse verbindbar. Auf diese Weise ist die Position der Schwenkführung zwischen dem Befestigungsstück und dem Handhabungsgehäuse eindeutig definiert.

Gemäß einer weiteren beispielhaften Ausgestaltung umfasst die Schwenkführung einen Führungszapfen und eine Führungsöffnung zur Aufnahme des Führungszapfens. Auf diese Weise ergibt sich eine axiale Fügebewegung (parallel zur Längsachse). Der Führungszapfen und die Führungsöffnung können relativ zueinander verschwenkt werden.

Gemäß einer weiteren beispielhaften Ausgestaltung ist die Führungsöffnung koaxial zur Längsachse in einem vom proximalen Ende des Schafts abgewandten Bereich in das Handhabungsgehäuse eingebracht. Diese Gestaltung bietet sich beispielsweise bei einem gekrümmten Handhabungsgehäuse an, weil dessen zweites Ende mit dem dortigen Leitungsanschluss aufgrund der Krümmung von der Längsachse beabstandet ist.

Gemäß einer weiteren beispielhaften Ausgestaltung weist die Schwenkführung eine zumindest abschnittsweise umlaufende Führungsnut und einen an die Führungsnut angepassten Haltering auf, der im montierten Zustand die Führungsnut zumindest abschnittsweise umgreift. Auf diese Weise kann sich eine radiale Fügebewegung senkrecht zur Längsachse ergeben.

Die Führungsnut und der Haltering können sich jeweils über 360° (Vollkreis) erstrecken. Auch geringere Bogenlängen von Kreissektoren (weniger als 360°) sind vorstellbar, wobei dies die freie Rotation zwischen dem Handhabungsgehäuse und dem Zusatzgriff, insbesondere dem Griffstück, gegebenenfalls entsprechend einschränkt.

Beispielhaft ist die Führungsnut als Zylinderfläche oder Zylinderabschnittsfläche gestaltet. Beispielhafte ist der Haltering als geschlossener Ring oder geöffneter Ring (Ringabschnitt) gestaltet. In einer beispielhaften Ausgestaltung kann der Haltering für die Montage und Demontage des Zusatzgriffs, insbesondere des Griffstücks, am Handhabungsgehäuse bedarfsweise geöffnet und geschlossen werden. Die Lagesicherung zwischen der Führungsnut in dem Haltering kann beispielsweise formschlüssig und/oder kraftschlüssig erfolgen.

Gemäß einer weiteren beispielhaften Ausgestaltung ist die Schwenkführung beim ersten Ende des Handhabungsgehäuses angeordnet. Dies hat den Vorteil, dass die Schwenkführung mit geringem Aufwand konzentrisch zum Schaft bzw. konzentrisch zur Längsachse ausgerichtet werden kann. Auf diese Weise können die Längsachse und die Schwenkachse deckungsgleich ausgerichtet sein. Die Anordnung beim ersten Ende des Handhabungsgehäuses kann eine Anordnung in einem ersten Endbereich des Handhabungsgehäuses umfassen.

Gemäß einer weiteren beispielhaften Ausgestaltung ist die Schwenkführung näher beim zweiten Ende des Handhabungsgehäuses als beim ersten Ende des Handhabungsgehäuses angeordnet. Dies umfasst beispielsweise eine Anordnung in einem Bereich von mehr als 50 % der Erstreckung zwischen dem ersten Ende und dem zweiten Ende des Handhabungsgehäuses.

Gemäß einer weiteren beispielhaften Ausgestaltung umfasst die Schwenkführung eine Kopplung des Zusatzgriffs, insbesondere des Griffstücks, und des Handhabungsgehäuses in einem Bereich, in dem ein Querschnitt des Handhabungsgehäuses senkrecht zur Längsachse einen Querschnitt der Schwenkführung einschließt. Mit anderen Worten kann die Schwenkführung relativ einfach am Umfang des Handhabungsgehäuse ausgebildet sein, weil genügend Raum für ein männliches Führungsteil und ein weibliches Führungsteil bereitsteht.

Der Querschnitt der Schwenkführung ist üblicherweise ein Kreis oder Ring. Der Querschnitt des Handhabungsgehäuses (ohne dass dort eine Schwenkführung eingebracht ist) kann von der reinen Kreisgestalt abweichen. Mit anderen Worten soll also der Querschnitt des Handhabungsgehäuses im Bereich der "Schnittstelle" genügend Raum zur Verfügung stellen, so dass dort die Schwenkführung eingebracht werden kann.

Gemäß einer weiteren beispielhaften Ausgestaltung umfasst die Schwenkführung eine Kopplung des Zusatzgriffs, insbesondere des Griffstücks, und des Handhabungsgehäuses in einem Bereich, in dem ein Querschnitt der Schwenkführung zumindest abschnittsweise außerhalb eines Querschnitts des Handhabungsgehäuses senkrecht zur Längsachse angeordnet ist.

Mit anderen Worten erfolgt dann die Kopplung in einem Bereich, in dem am Handhabungsgehäuse grundsätzlich gar nicht genug Raum für die angestrebte konzentrische Ausrichtung zwischen Schwenkachse und Längsachse zur Verfügung steht. Grundsätzlich kann auch in einem solchen Bereich die Schnittstelle mit Schwenkführung zwischen dem Handhabungsgehäuse und dem Zusatzgriff, insbesondere dem Griffstück, ausgebildet sein, wenn man gegebenenfalls einen Axialversatz zwischen der Schwenkachse und der Längsachse in Kauf nimmt. Es ist jedoch auch vorstellbar, über ein Zwischenstück (beispielsweise Befestigungsstück) die gewünschte konzentrischer Ausrichtung zwischen Schwenkachse und Längsachse herbeizuführen. Dies ist auch dann vorstellbar, wenn die Größe und Position des Querschnitts des Handhabungsgehäuses an dieser Schnittstelle eine solche Ausrichtung konstruktiv eigentlich nicht ermöglicht.

Gemäß einer weiteren beispielhaften Ausgestaltung ist die Schwenkachse koaxial zur Längsachse. Auf diese Weise wird während der Schwenkbewegung ein Wandern der Längsachse in Bezug auf die Schwenkachse verhindert.

Gemäß einer weiteren beispielhaften Ausgestaltung ist die Schwenkachse parallel und versetzt zur Längsachse orientiert. Auf diese Weise kann auch bei einem gekrümmten Handhabungsgehäuse eine Schwenkführung ohne Zwischenteile bereitgestellt werden, wenn nutzerseitig während der Schwenkbewegung das Wandern (Umlaufen) der Längsachse in Bezug auf die Schwenkachse akzeptiert wird.

Gemäß einer weiteren beispielhaften Ausgestaltung sind der Zusatzgriff, insbesondere das Griffstück, und das Handhabungsgehäuse im montierten Zustand des Handhabungsgehäuses relativ zueinander um eine Kippachse verschwenkbar, die im Wesentlichen senkrecht zur Längsachse des Schafts orientiert ist. Mit anderen Worten gibt es einen zusätzlichen Freiheitsgrad für den Zusatzgriff, insbesondere das Griffstück. Die Neigung zwischen dem Zusatzgriff, insbesondere dem Griffstück, und der Längsachse kann verändert werden. Dies umfasst beispielsweise einen Neigungswinkel (Kippwinkel) zwischen einer 0°-Orientierung (Zusatzgriff, insbesondere Griffstück, ist in Verlängerung des Handhabungsgehäuses etwa parallel zur Längsachse ausgerichtet) und einer 90°-Orientierung (Zusatzgriff, insbesondere Griffstück, ist etwa senkrecht zur Längsachse ausgerichtet), wobei auch Zwischenpositionen vorstellbar sind. Andere Bereiche und Zwischenpositionen für den Neigungswinkel sind denkbar. In einer beispielhaften Ausgestaltung ist die Kippachse senkrecht zur Längsachse des Schafts orientiert.

Gemäß einer weiteren beispielhaften Ausgestaltung ist die Kippachse durch eine Kippführung definiert, die außerhalb des Handhabungsgehäuses ausgebildet ist. Insbesondere ist die Kippführung beim Zusatzgriff, insbesondere beim Griffstück, ausgebildet. Auf diese Weise kann das Handhabungsgehäuse weiterhin kompakt gestaltet sein. Gemäß einer weiteren beispielhaften Ausgestaltung wird die Kippführung durch ein Drehgelenk oder Kugelgelenk bereitgestellt.

Gemäß einer weiteren beispielhaften Ausgestaltung ist eine Schwenkbewegung um die Schwenkachse indexiert. Gemäß einer weiteren beispielhaften Ausgestaltung ist ein Drehraster für die Schwenkbewegung verbaut. Auf diese Weise kann die Schwenkbewegung eine definierte inkrementale Bewegung mit bestimmter Schrittweite umfassen. Durch geeignete Rastgeometrien (Verzahnung und dergleichen) kann eine Rückmeldung an den Nutzer erfolgen, beispielsweise eine haptische oder akustische Rückmeldung.

Gemäß einer weiteren beispielhaften Ausgestaltung ist der Zusatzgriff, insbesondere das Griffstück, im montierten Zustand mittelbar oder unmittelbar unter Vorspannung spielarm am oder relativ zum Handhabungsgehäuse geführt. Auf diese Weise kann mit nur wenigen Bauteilen ein fester und sicherer Halt zwischen dem Zusatzgriff, insbesondere dem Griffstück, und dem Handhabungsgehäuse bereitgestellt werden, obwohl dort ein Freiheitsgrad für die Schwenkbewegung vorgesehen ist.

Gemäß einer weiteren beispielhaften Ausgestaltung weist das Instrument ferner einen Lagesensor auf, der insbesondere als Beschleunigungssensor gestaltet ist, wobei der Lagesensor dazu ausgebildet ist, eine Drehorientierung des Instruments zu erfassen, auf deren Basis eine elektronische Bildaufrichtung von mit dem Instrument erfassten Aufnahmebildern ermöglicht ist. Beschleunigungssensoren können auch als Trägheitssensoren bezeichnet werden. Es versteht sich, dass auch andere Sensoren als Beschleunigungssensoren verbaut sein können. Der Lagesensor ist beispielhaft im Handhabungsgehäuse und/oder im Schaft des Instruments verbaut. Die elektronische Bildaufrichtung bei einem endoskopischen Instrument unter Nutzung von Trägheitssensoren ist beispielsweise in der US 7,037,258 B2 beschrieben.

In einer beispielhaften Ausgestaltung wird das vom Lagesensor erfasste Signal genutzt, um das erfasste Bild in Bezug auf einen Referenzhorizont auszurichten. Mit anderen Worten soll sich der Horizont des Bildes auch während der Rotation des Instruments um die Längsachse nicht ändern. Auf diese Weise kann für einen Benutzer ein ähnlicher Effekt wie bei einer Pendelkamera bereitgestellt werden, die sich im Gegensatz zum Bildaufnehmer im Schaft des Instruments nicht mit dem Instrument verdreht. Wenn also bestimmte Nutzergruppen einen konstanten Horizont wie bei einer Pendelkamera wünschen, kann dies unter Nutzung des vom Lagesensor erfassten Signals durch entsprechende Ausrichtung der erfassten Bilder bereitgestellt werden.

Gemäß einer weiteren beispielhaften Ausgestaltung ist das Handhabungsgehäuse aus einer Metalllegierung, insbesondere einer Leichtmetallregierung, gefertigt. Gemäß einer weiteren beispielhaften Ausgestaltung ist der Zusatzgriff, insbesondere das Griffstück, aus einem thermoplastischen Kunststoff, insbesondere einem hochtemperaturbeständigen Kunststoff, gefertigt. Beispielhaft handelt es sich bei dem Kunststoff um einen PEEK oder einen vergleichbaren Hochleistungskunststoff. Beispielhaft handelt es sich bei der Metalllegierung des Handhabungsgehäuses um eine Aluminiumlegierung, insbesondere um eloxiertes Aluminium.

Gemäß einem weiteren Aspekt bezieht sich die vorliegende Offenbarung auf ein chirurgisches System mit einem Instrument gemäß zumindest einer der hierin beschriebenen Ausgestaltungen, ferner aufweisend einen dem Handhabungsgehäuse vorgelagerten Handhabungsabschnitt mit zumindest einer Handhabe zur Steuerung eines Behandlungsvorgangs, insbesondere zur Behandlung von Gewebe und/oder Ablagerungen im Körperinneren eines menschlichen oder tierischen Patienten. Es kann vorgesehen sein, dass der Handhabungsabschnitt gemeinsam mit dem Handhabungsgehäuse relativ zum Zusatzgriff, insbesondere zum Griffstück, um die Schwenkachse verschwenkbar ist.

Ein solches System ist beispielsweise als Resektoskop oder als anderes chirurgisches Instrument gestaltet, die sowohl zur Beobachtung als auch zur Behandlung von Gewebe und dergleichen dienen. Resektoskope und vergleichbare Instrumente erfordern während der chirurgischen Prozedur regelmäßig eine Rotation des Instruments, um das Instrument in geeigneter Weise in Bezug auf zu beobachtendes und/oder zu behandelndes Gewebe zu positionieren.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Offenbarung zu verlassen.

Weitere Merkmale und Vorteile der Erfindung ergeben sich aus der nachfolgenden Beschreibung und Erläuterung mehrerer beispielhafter Ausführungsformen unter Bezugnahme auf die Zeichnungen. Es zeigen:
- Fig. 1:: eine seitliche Ansicht eines Resektoskopes mit konventionellem Endoskop und Kamerakopf;
- Fig. 2:: eine seitliche Ansicht eines Resektoskopes mit einem Chip-on-the-Tip Endoskop;
- Fig. 3:: eine seitliche Ansicht eines Instruments mit distalem Bildaufnehmer in einer ersten Orientierung;
- Fig. 4:: eine weitere Ansicht des Instruments gemäß Fig. 3 in einer um 90° um eine Längsachse des Instruments verschwenkten Orientierung;
- Fig. 5:: eine seitliche Teilansicht einer Ausführungsform eines Instruments mit einem an ein Handhabungsgehäuse angekoppelten Zusatzgriff, insbesondere Griffstück;
- Fig. 6:: eine vergrößerte, teilweise geschnittene Detailansicht der Anordnung gemäß Fig. 5 im Bereich einer Schnittstelle zwischen dem Griffstück und dem Handhabungsgehäuse;
- Fig. 7: eine seitliche, leicht perspektivische Teilansicht einer weiteren Ausführungsform eines Instruments mit einem an ein Handhabungsgehäuse angekoppelten Griffstück;
- Fig. 8: eine vergrößerte, teilweise geschnittene Detailansicht der Anordnung gemäß Fig. 7 im Bereich einer Schnittstelle zwischen dem Griffstück und dem Handhabungsgehäuse;
- Fig. 9: eine seitliche Teilansicht einer weiteren Ausführungsform eines Instruments mit einem an ein Handhabungsgehäuse angekoppelten Griffstück;
- Fig. 10: eine vergrößerte, teilweise geschnittene Detailansicht der Anordnung gemäß Fig. 9 im Bereich einer Schnittstelle zwischen dem Griffstück und dem Handhabungsgehäuse;
- Fig. 11: eine seitliche Teilansicht einer weiteren Ausführungsform eines Instruments mit einem an ein Handhabungsgehäuse angekoppelten Griffstück;
- Fig. 12: eine vergrößerte, teilweise geschnittene Detailansicht der Anordnung gemäß Fig. 11 im Bereich einer Schnittstelle zwischen dem Griffstück und dem Handhabungsgehäuse;
- Fig. 13: eine weitere, auf der Anordnung gemäß Fig. 11 beruhende Ausführungsform eines Instruments mit einem an ein Handhabungsgehäuse angekoppelten Griffstück;
- Fig. 14: eine auf Fig. 13 beruhende vergrößerte Schnittansicht durch das Griffstück im Bereich einer Kippführung, wobei die Schnittebene parallel zur Schwenkachse und parallel zur Kippachse ist; und
- Fig. 15:: eine schematische, teilweise geschnittene Detailansicht einer Schnittstelle zwischen einem Handhabungsgehäuse und einem Griffstück zur Veranschaulichung eines Drehrasters.

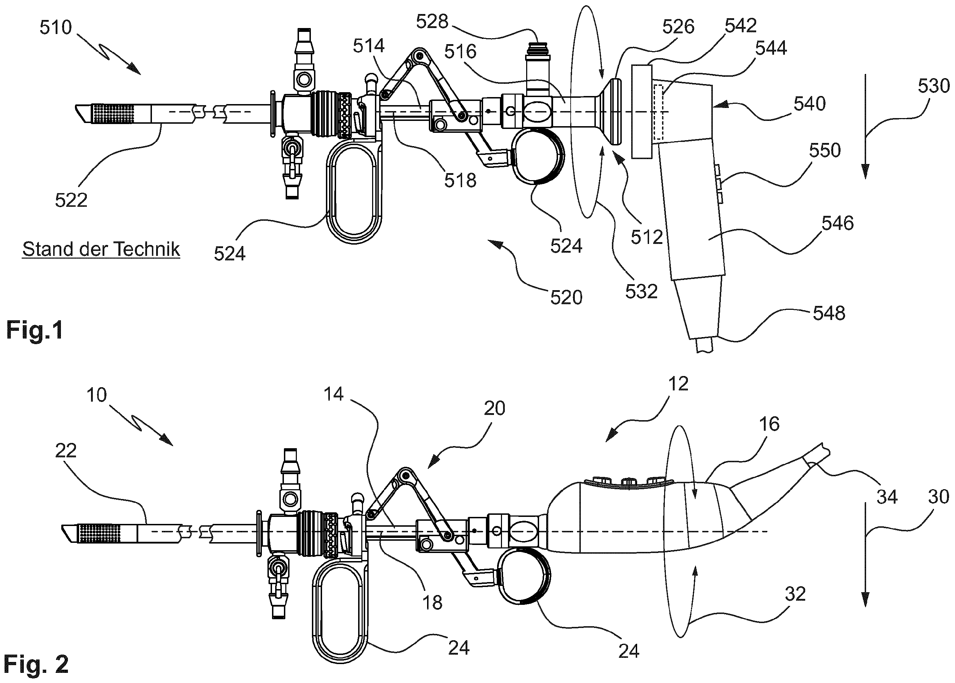

Fig. 1 veranschaulicht anhand einer seitlichen Ansicht ein grundsätzlich aus dem Stand der Technik bekanntes chirurgisches System 510, das hier als Resektoskop gestaltet ist. Das System 510 umfasst ein Instrument 512, das als endoskopisches Beobachtungsinstrument gestaltet ist. Das Instrument 512 umfasst einen Schaft 514 und einen Kopf 516 an einem proximalen Ende des Schafts 512. Der Schaft 514 definiert entlang seiner Längserstreckung eine Längsachse 518.

Das System 510 umfasst ferner einen Handhabungsabschnitt 520 mit einem äußeren Schaft 522 und zumindest einer Handhabe 524. Der Schaft 514 erstreckt sich zumindest teilweise durch den äußeren Schaft 522. Bei der Gestaltung als Resektoskop dient der Handhabungsabschnitt 520 beispielsweise zur Steuerung und Aktivierung von Elektroden. Das insoweit konventionell (rein optisch) gestaltete Instrument 512 weist an seinem proximalen Ende beim Kopf 516 ein Okular 526 auf. Ferner weist der Kopf 516 einen Anschluss 528 auf, der beispielsweise zum Anschluss eines Lichtleiters dient.

In Fig. 1 kennzeichnet ein mit 530 bezeichneter Pfeil als beispielhafte Referenz die Richtung der Schwerkraft. Während der chirurgischen Prozedur kann es erforderlich sein, das Resektoskop (System 510) mit dem Instrument 512 um die Längsachse 518 zu verschwenken, vergleiche hierzu einen mit 532 bezeichneten Doppelpfeil. Auf diese Weise kann das Resektoskop günstig in Bezug auf einen gewünschten Bereich (Region of Interest) im Körperinneren positioniert werden. Dies ist insbesondere bei einer Gestaltung des Instruments 512 als Schrägblick-Instrument häufig angezeigt.

Ferner zeigt Fig. 1 einen schematisch angedeuteten Kamerakopf 540, der über ein Montagestück 542 mit dem Okular 526 des Instruments 512 koppelbar ist. Der Kamerakopf 540 ist beispielhaft als Pendelkamera gestaltet. Der Kamerakopf 540 weist einen Bildaufnehmer 544 zur Erfassung einer am Okular 526 bereitgestellten Abbildung auf. Auf diese Weise kann ein Videosignal erzeugt werden. Der Kamerakopf 45 umfasst ferner ein Griffstück 546, das etwa als Pistolengriff gestaltet und um einen stumpfen Winkel gegenüber der Längsachse 518 geneigt ist. An seinem vom Montagestück 542 abgewandten Ende weist der Kamerakopf 540 einen Leitungsanschluss 548 zur Ankopplung einer Versorgungsleitung auf. Ferner sind beim Griffstück 546 Betätigungselemente 550 zur Steuerung des Kamerakopfes 540 angeordnet.

Ein Bediener kann also im gekoppelten Zustand das Instrument 512 bzw. das Resektoskop (System 510) über den Kamerakopf 540 mit dem Griffstück 546 halten und führen. Das Instrument 512 ist um die Längsachse 518 relativ zum Kamerakopf 540 verschwenkbar. Es findet dann eine Relativverschwenkung zwischen dem Instrument 512 und dem Kamerakopf 540 statt. Es hat sich gezeigt, dass viele Anwender diese Gestaltung als ergonomischen günstig betrachten. Der Kamerakopf 540 mit dem Griffstück 546 dient gewissermaßen als Basis oder Referenz für die Rotationsbewegung des Instruments 512. Ein Bediener kann das gesamte System 510 mit zwei Händen gut halten, führen und betätigen, dies umfasst fallweise auch die Schwenkbewegung um die Längsachse 518.

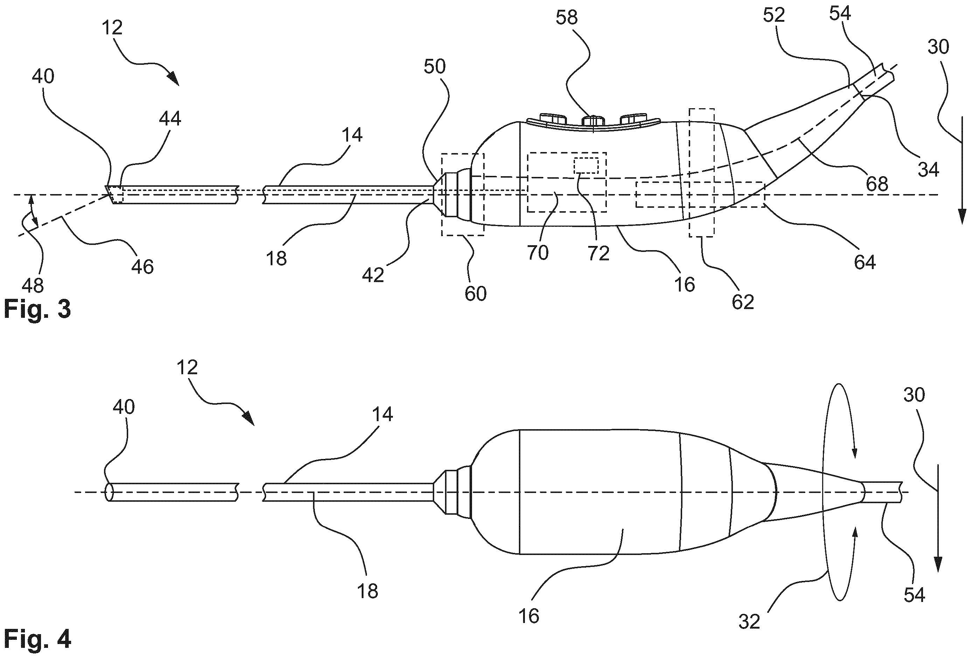

Mit Bezugnahme auf Fig. 2 sowie mit ergänzender Bezugnahme auf die Figuren 3 und 4 wird eine beispielhafte Ausgestaltung eines Instruments 12 veranschaulicht, das beispielhaft Bestandteil eines als Resektoskop gestalteten Systems 10 ist. Das Instrument 12 weist einen Schaft 14 auf, der eine Längsachse 18 definiert. An den Schaft 14 schließt sich ein Handhabungsgehäuse 16 an, das im Ausführungsbeispiel zumindest teilweise als Verlängerung des Schafts 14 gestaltet ist. Das Instrument 12 ist beispielsweise als Videoendoskop mit integriertem Bildaufnehmer gestaltet.

Das System umfasst ferner einen Handhabungsabschnitt 20 mit einem äußeren Schaft 22, durch den sich der Schaft 14 zumindest teilweise erstreckt. Der Handhabungsabschnitt 20 umfasst eine oder mehrere Handhaben 24, die beispielhaft zur Steuerung von Elektroden dienen. Es versteht sich, dass das System 10 nicht notwendigerweise ein Resektoskop sein muss, die vorliegende Offenbarung ist in dieser Hinsicht nicht einschränkend zu verstehen.

Ähnlich wie in Fig. 1 dient in den Figuren 2-4 ein mit 30 bezeichneter Pfeil aus Veranschaulichungsgründen als Richtungsreferenz. Der Pfeil 30 veranschaulicht die Richtung der Schwerkraft. Es versteht sich, dass das Instrument 12 auch anders als in den Figuren 2-4 in Bezug auf die Richtung der Schwerkraft ausgerichtet sein kann.

Während der chirurgischen Prozedur kann es erforderlich sein, dass ein Bediener das System 10 bzw. das Instrument 12 um die Längsachse 18 rotiert. Vergleiche hierzu einen mit 32 bezeichneten gekrümmten Doppelpfeil. Eine solche Bewegung erlaubt bei einem Schrägblick-Instrument eine Veränderung des Sichtfeldes. Im Gegensatz zur Anordnung gemäß Fig. 1 erfolgt bei der Anordnung gemäß den Figuren 2-4 keine Bewegung des Instruments 12 in Bezug auf eine körperliche Referenz (Kamerakopf oder dergleichen). Dies hat ferner zur Folge, dass etwa auch ein Leitungsanschluss 34 an einem proximalen Ende des Handhabungsgehäuses 16 gemeinsam mit dem Instrument 12 rotiert wird, im Gegensatz zum Leitungsanschluss 548 bei der Anordnung gemäß Fig. 1.

Die Figuren 3 und 4 veranschaulichen die weitere Gestaltung des Instruments 12 gemäß Fig. 2. In Fig. 4 ist das Instrument 12 gegenüber der Orientierung in Fig. 3 um 90° um die Längsachse 18 rotiert, vergleiche die jeweilige Referenz durch den Pfeil 30 (Richtung der Schwerkraft). Der Schaft 14 erstreckt sich zwischen einem distalen Ende 40 und einem proximalen Ende 42. In einem distalen Endbereich des Schafts 14 ist ein Bildaufnehmer 44 angeordnet. Der Bildaufnehmer 44 ist bei dem distalen Ende 40 des Schafts 14 angeordnet. Die Bildaufnehmer 44 kann distal eine Optik und/oder ein Schutzfenster vorgelagert sein. In Fig. 3 ist mit 46 eine Blickrichtung des Instruments 12 angedeutet. Ein mit 48 bezeichneter Winkel veranschaulicht die Neigung der Blickrichtung gegenüber der Längsachse 18. Der Winkel 48 beträgt beispielhaft 30°, andere Werte sind vorstellbar, umfassend etwa 25°, 45°, 60°, 90° oder dergleichen. Grundsätzlich ist auch eine Gestaltung des Instruments 12 als Instrument mit gerader Blickrichtung (Winkel 48 beträgt 0°) vorstellbar. Insbesondere bei der Gestaltung als Schrägblick-Instrument erlaubt eine Schwenkbewegung des Instruments 12 um die Längsachse 18 ein Wandern (Umlaufen) des Sichtfeldes, so dass insgesamt ein größerer Bereich observiert werden kann.

Das Handhabungsgehäuse 16 erstreckt sich zwischen einem ersten Ende 50 und einem zweiten Ende 52. Das erste Ende 50 kann auch als distales Ende bezeichnet werden. Das zweite Ende 52 kann auch als proximales Ende bezeichnet werden. Das Handhabungsgehäuse 16 schließt sich an das proximale Ende 42 des Schafts 14 an. Im Ausführungsbeispiel sind das Handhabungsgehäuse 16 und der Schaft 14 drehfest miteinander verbunden. Demgemäß gibt es keine Relativrotation zwischen dem Handhabungsgehäuse 16 und dem Schaft 14 um die Längsachse 18. Beim zweiten Ende 52 des Handhabungsgehäuses 16 ist der Leitungsanschluss 34 ausgebildet, an denen eine Leitung 54 ankoppelt. Die Leitung 54 ist eine Versorgungsleitung. Beispielhaft dient die Leitung 54 zur Energieversorgung und/oder zur Datenübertragung. Das Handhabungsgehäuse 16 beherbergt Betätigungselemente 58, beispielsweise in Form von Knöpfen, Tastern und ähnlichem. Es handelt sich dabei insbesondere um Schalter zur Steuerung elektronischer/elektrischer Funktionen.

Die Darstellung gemäß Fig. 3 veranschaulicht anhand gestrichelter Blöcke denkbare Bereiche für Aufnahmen 60, 62, 64 zur Kopplung des Handhabungsgehäuses 16 mit einem Griffstück. Die Aufnahme 60 ist beim ersten Ende 50 des Handhabungsgehäuses 16 angeordnet. Die Aufnahmen 62, 64 sind näher beim zweiten Ende 52 als beim ersten Ende 50 angeordnet.

Den Figuren 3 und 4 ist ferner entnehmbar, dass das Handhabungsgehäuse 16 im Ausführungsbeispiel eine gekrümmte Haupterstreckungsrichtung 68 aufweist. Mit anderen Worten ist die Haupterstreckungsrichtung 68 des Handhabungsgehäuses 16 nicht parallel zur Längsachse 18. Die Haupterstreckungsrichtung 68 ist beispielsweise durch die jeweiligen geometrischen Schwerpunkte der Querschnitte des Handhabungsgehäuses 16 entlang der Erstreckung des Handhabungsgehäuses 16 definiert. Hin zu seinem zweiten Ende 52 ist das Handhabungsgehäuse 16 deutlich gegenüber der Längsachse 18 geneigt. Insgesamt ist das Handhabungsgehäuse 16 beispielhaft ähnlich einer halben Banane gestaltet, wobei der Leitungsanschluss 34 beim Stängel angeordnet ist. Die teilweise gekrümmte Gestaltung des Handhabungsgehäuses 16 kann ergonomisch etwa dann von Vorteil sein, wenn das Instrument 12 über das Handhabungsgehäuse 16 ähnlich einem Stylus gehalten und geführt wird.

Ferner ist zu beachten, dass das Handhabungsgehäuse 16 überwiegend nicht rotationssymmetrisch zur Längsachse 18 gestaltet ist. Auch dies erschwert gegebenenfalls die Integration eines Griffstücks.

Das Handhabungsgehäuse 16 beherbergt ferner Steuerungskomponenten, insbesondere elektronische Steuerungskomponenten zur Steuerung des Instruments 12. Beispielhaft ist in Fig. 3 schematisch eine Steuereinheit 70 innerhalb des Handhabungsgehäuses 16 angedeutet. In beispielhaften Ausgestaltungen beherbergt das Handhabungsgehäuse 16 zumindest einen Lagesensor 72, der eine Erfassung der Position und/oder Orientierung des Instruments 12 ermöglicht. Der Lagesensor 72 kann grundsätzlich im Handhabungsgehäuse 16 verbaut sein, alternativ ist auch eine Anordnung im Schaft 14 vorstellbar. Beispielhaft ist der Lagesensor 72 als Trägheitssensor gestaltet. Mit dem Lagesensor 72 können Schwenkbewegungen (Rotationsbewegungen) 32 um die Längsachse 18 erfasst werden. Auf diese Weise kann beispielhaft eine elektronisch Bildaufrichtung erfolgen, wenn vom Bediener auch während der Rotationsbewegung des Instruments 12 ein einheitlicher Horizont bei der Beobachtung gewünscht ist. Auf diese Weise kann das Instrument 12 bzw. ein damit versehenes System 10 (vergleiche Fig. 2) bei der Schwenkbewegung 32 ein Verhalten an den Tag legen, das dem Verhalten des mit einem konventionellen Beobachtungsinstrument 512 versehenen Systems 510 (vergleiche Fig. 1) ähnelt.

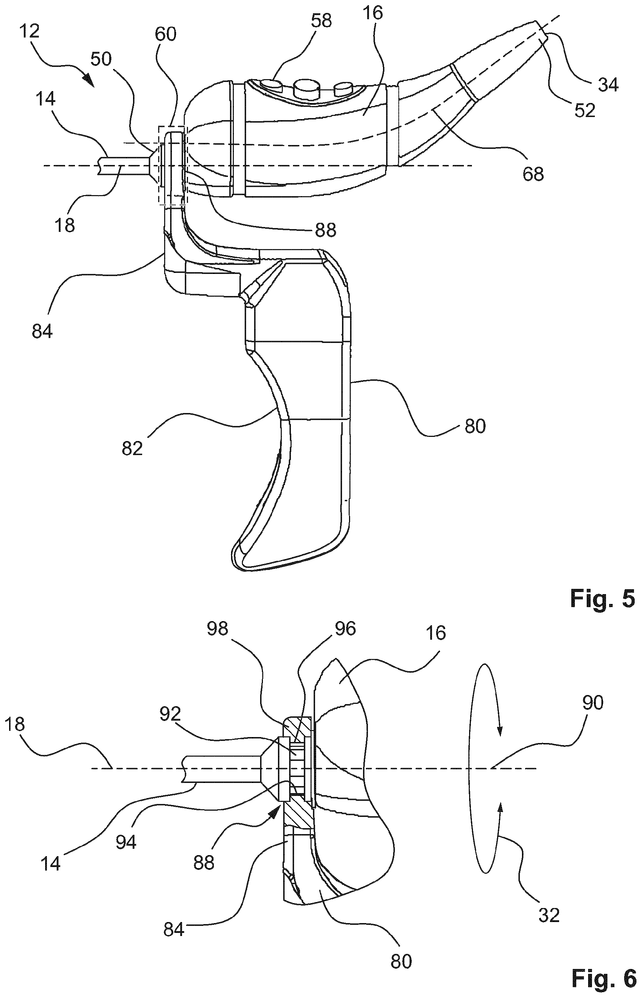

Mit Bezugnahme auf die Figuren 5-15 werden verschiedene Ausgestaltungen von Zusatzgriffen, insbesondere Griffstücken 80, 180, 280, 380, und deren Kopplung mit Handhabungsgehäusen 16 einschlägiger Instrumente 12 zur Ausbildung einer jeweiligen Schwenkführung 88, 188, 288, 388 veranschaulicht. Hinsichtlich der grundsätzlichen Gestaltung des Instruments 12 wird zur Vermeidung von Wiederholungen auf die vorstehenden Ausführungen in Zusammenhang mit den Figuren 2-4 verwiesen. Die Griffstücke 80, 180, 280, 380 sind abnehmbar.

Die Griffstücke 80, 180, 280, 380 können alternativ drehfest mit dem Handhabungsgehäuse 16 verbunden sein. Ferner können die Griffstücke 80, 180, 280, 380 alternativ auch einstückig mit dem Handhabungsgehäuse 16 ausgebildet sein.

Die nachfolgend veranschaulichten Griffstücken 80, 180, 280, 380 sind in beispielhaften Ausgestaltungen als Zusatzgriffe des jeweiligen Instruments 12 gestaltet. Mit anderen Worten können die Instrument 12 grundsätzlich auch ohne die Griffstücke 80, 180, 280, 380 betrieben und genutzt werden. Die Griffstücke 80, 180, 280, 380 erlauben jedoch eine günstige Handhabung und Positionierung der Instrument 12, zumindest für bestimmte Anwendungen. Dies betrifft beispielhaft solche Anwendungen, bei denen häufig zur Ausrichtung eine Relativrotation der Instrumente 12 um die Längsachse 18 erforderlich ist.

Mit Bezugnahme auf die Figuren 5 und 6 wird eine beispielhafte Ausgestaltung eines Griffstücks 80 veranschaulicht, das in einem Bereich (vergleiche Bezugszeichen 60 in Fig. 3) beim ersten Ende 50 mit dem Handhabungsgehäuse 16 des Instruments 12 koppelbar ist. Das Griffstück 80 weist beispielhaft eine Fingermulde oder Griffmulde 82 auf. Das Griffstück 80 ist über einen Ausleger 84 unter Ausbildung einer Schwenkführung 88 mit dem Handhabungsgehäuse 16 gekoppelt. In Fig. 5 ist das Griffstück 80 etwa senkrecht zur Längsachse 18 ausgerichtet. Der Ausleger 84 ist im Ausführungsbeispiel etwa L-förmig gestaltet, ein mit dem Griffstück 80 verbundener Schenkel ist etwa parallel zur Längsachse 18 und ein mit dem Handhabungsgehäuse 16 gekoppelter Schenkel ist etwa senkrecht zur Längsachse 18. Dies ist nicht einschränkend zu verstehen.

Die vergrößerte, teilweise geschnittene Darstellung gemäß Fig. 6 veranschaulicht, dass die Schwenkführung 88 konzentrisch zur Längsachse 18 durch den Schaft 14 orientiert ist. Im Ergebnis erfolgt die Schwenkbewegung 32 um eine Schwenkachse 90, die deckungsgleich zur Längsachse 18 ist. Die Schwenkführung 88 umfasst ein männliches Führungsteil 92 und ein weibliches Führungsteil 94, die jeweils einen kreisförmigen Querschnitt aufweisen. Im Ausführungsbeispiel ist das männliche Führungsteil 92 als Führungsnut 96 ausgebildet, die am Handhabungsgehäuse 16 im Bereich des ersten Endes 50 umläuft. Im Ausführungsbeispiel ist das weibliche Führungsteil 94 als Haltering 98 ausgebildet, der beim Ausleger 84 des Griffstücks 80 ausgebildet ist.

Das Griffstück 80 kann kraftschlüssig und/oder formschlüssig mit dem Handhabungsgehäuse 16 des Instruments 12 gefügt werden. Eine gewisse Vorspannung (Haltemoment) für den Sitz zwischen dem Griffstück 80 und dem Instrument 12 bei der Schwenkführung 88 ist durchaus gewünscht, um eine unbeabsichtigte Verdrehung zu vermeiden. Gleichwohl soll die Schwenkführung 88 gerade keine drehfest/drehsteife Verbindung umfassen, damit der Benutzer bei der Bedienung des Instruments 12 die gewünschte Schwenkbewegung 32 einfach ausführen kann, während das Instrument 12 über das Griffstück 80 gehalten wird.

Bei der in den Figuren 5 und 6 gezeigten Gestaltung mit dem Griffstück 80 erlaubt die Schwenkführung 88 eine Übereinstimmung zwischen der Schwenkachse 90 und der Längsachse 18, auch wenn die Haupterstreckungsrichtung 68 (vergleiche Fig. 5) des Handhabungsgehäuses 16 überwiegend deutlich von der Längsachse 18 beabstandet ist.

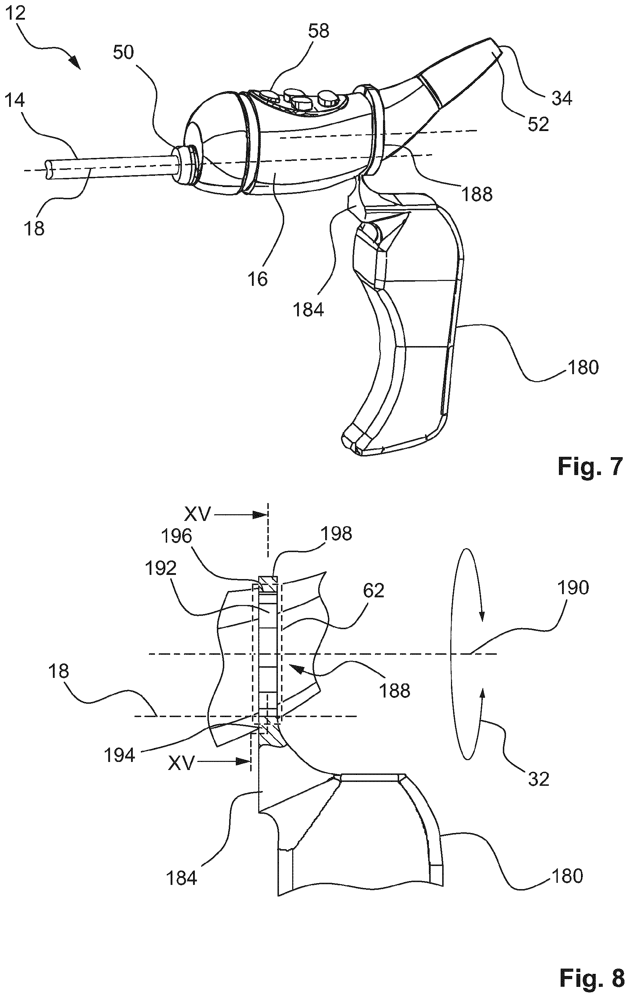

Mit Bezugnahme auf die Figuren 7 und 8 wird eine weitere beispielhafte Ausgestaltung eines Griffstücks 180 veranschaulicht, das in einem Bereich (vergleiche Bezugszeichen 62 in Fig. 3), der näher beim zweiten Ende 52 als beim ersten Ende 50 des Handhabungsgehäuses 16 liegt, mit dem Handhabungsgehäuse 16 des Instruments 12 koppelbar ist. Das Griffstück 180 ist über einen Ausleger 184 unter Ausbildung einer Schwenkführung 188 mit dem Handhabungsgehäuse 16 gekoppelt. In Fig. 7 ist das Griffstück 180 etwa senkrecht zur Längsachse 18 ausgerichtet. Der Ausleger 184 ist im Ausführungsbeispiel etwa L-förmig gestaltet, ein mit dem Griffstück 180 verbundener Schenkel ist etwa parallel zur Längsachse 18 und ein mit dem Handhabungsgehäuse 16 gekoppelter Schenkel ist etwa senkrecht zur Längsachse 18. Dies ist nicht einschränkend zu verstehen.

Die vergrößerte, teilweise geschnittene Darstellung gemäß Fig. 8 veranschaulicht, dass die Schwenkführung 188 nicht konzentrisch zur Längsachse 18 durch den Schaft 14 orientiert ist. Im Ergebnis erfolgt die Schwenkbewegung 32 im Ausführungsbeispiel um eine Schwenkachse 190, die parallel und versetzt zur Längsachse 18 ist. Die Schwenkführung 188 umfasst ein männliches Führungsteil 192 und ein weibliches Führungsteil 194, die jeweils einen kreisförmigen Querschnitt aufweisen. Im Ausführungsbeispiel ist das männliche Führungsteil 192 als Führungsnut 96 ausgebildet, die am Handhabungsgehäuse 16 umläuft. Im Ausführungsbeispiel ist das weibliche Führungsteil 194 als Haltering 198 ausgebildet, der beim Ausleger 184 des Griffstücks 180 ausgebildet ist.

Das Griffstück 180 kann kraftschlüssig und/oder formschlüssig mit dem Handhabungsgehäuse 16 des Instruments 12 gefügt werden. Die Kopplung soll einerseits hinreichend fest und genau sein, andererseits eine einhändige Verstellung ermöglichen, wenn das Instrument 12 mit dem Handhabungsgehäuse 16 um die Schwenkachse 190 relativ zum Griffstück 180 rotiert wird.

Bei der in den Figuren 7 und 8 gezeigten Gestaltung mit dem Griffstück 180 erfolgt die Schwenkführung 188 um eine Schwenkachse 190, die gegenüber der Längsachse 18 versetzt ist. Dies führt aus Sicht eines Bedieners neben der Schwenkbewegung (Rotation) zu einer Umlaufbewegung des Schafts 14 des Instruments 12 um die Schwenkachse 190, der Radius dieser Umlaufbewegung entspricht dem Abstand zwischen der Längsachse 18 und der Schwenkachse 190. Gegebenenfalls können das Sichtfeld und der Blickwinkel des Instruments 12 an diesen Versatz angepasst werden, so dass während einer Schwenkbewegung um 360° kein blinder Fleck im Zentrum verbleibt.

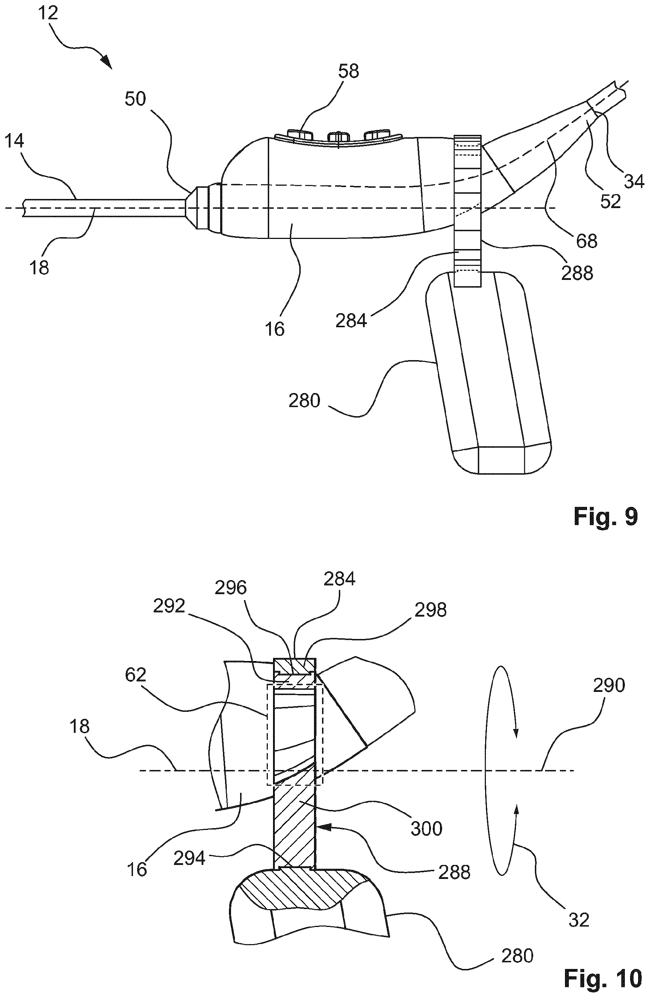

Mit Bezugnahme auf die Figuren 9 und 10 wird eine beispielhafte Ausgestaltung eines Griffstücks 280 veranschaulicht, das in einem Bereich (vergleiche Bezugszeichen 62 in Fig. 3), der näher beim zweiten Ende 52 als beim ersten Ende 50 des Handhabungsgehäuses 16 liegt, mit dem Handhabungsgehäuse 16 des Instruments 12 koppelbar ist. Insoweit ähnelt das Ausführungsbeispiel gemäß den Figuren 9 und 10 dem Ausführungsbeispiel gemäß den Figuren 7 und 8. Das Griffstück 280 ist über einen Ausleger 284 unter Ausbildung einer Schwenkführung 288 mit dem Handhabungsgehäuse 16 gekoppelt. In Fig. 9 ist das Griffstück 80 etwa senkrecht zur Längsachse 18 ausgerichtet bzw. leicht nach proximal geneigt. Im Ausführungsbeispiel ist also zwischen dem Griffstück 280 und der Längsachse 18 ein Winkel von beispielsweise 75° bis 85° ausgebildet, der nach proximal geöffnet ist. Dies ist nicht einschränkend zu verstehen.

Die vergrößerte, teilweise geschnittene Darstellung gemäß Fig. 10 veranschaulicht, dass die Schwenkführung 288 konzentrisch zur Längsachse 18 durch den Schaft 14 orientiert ist. Dies wird beim Ausführungsbeispiel gemäß den Figuren 9 und 10 durch ein Befestigungsstück 300 ermöglicht, dass gewissermaßen als Adapterstück zwischen dem Handhabungsgehäuse 16 und dem Griffstück 280 bzw. dessen Ausleger 284 agiert.

Im Ergebnis erfolgt die Schwenkbewegung 32 um eine Schwenkachse 290, die deckungsgleich zur Längsachse 18 ist. Die Schwenkführung 288 umfasst ein männliches Führungsteil 292, das durch das Befestigungsstück 300 bereitgestellt wird, und ein weibliches Führungsteil 294, die jeweils einen kreisförmigen Querschnitt aufweisen. Im Ausführungsbeispiel ist das männliches Führungsteil 292 als Führungsnut 296 ausgebildet, die am Befestigungsstück 300 umläuft. Im Ausführungsbeispiel ist das weibliche Führungsteil 294 als Haltering 298 ausgebildet, der beim Ausleger 284 des Griffstücks 280 ausgebildet ist. Das Befestigungsstück 300 ist im Bereich einer geeigneten Aufnahme (vergleiche Bezugszeichen 62 in Fig. 10) drehfest mit dem Handhabungsgehäuse 16 gekoppelt.

Das Griffstück 280 kann mittelbar über das Befestigungsstück 300 kraftschlüssig und/oder formschlüssig mit dem Handhabungsgehäuse 16 des Instruments 12 gefügt werden. Bei der in den Figuren 9 und 10 gezeigten Gestaltung mit dem Griffstück 280 erlaubt die Schwenkführung 288 eine Übereinstimmung zwischen der Schwenkachse 290 und der Längsachse 18, auch wenn die Haupterstreckungsrichtung 68 (vergleiche Fig. 9) des Handhabungsgehäuses 16 überwiegend deutlich von der Längsachse 18 beabstandet ist. Es gibt also bei der Schwenkbewegung 32 neben der Rotation des Schafts 14 um die Schwenkachse 290 keine überlagernde Umlaufbewegung.

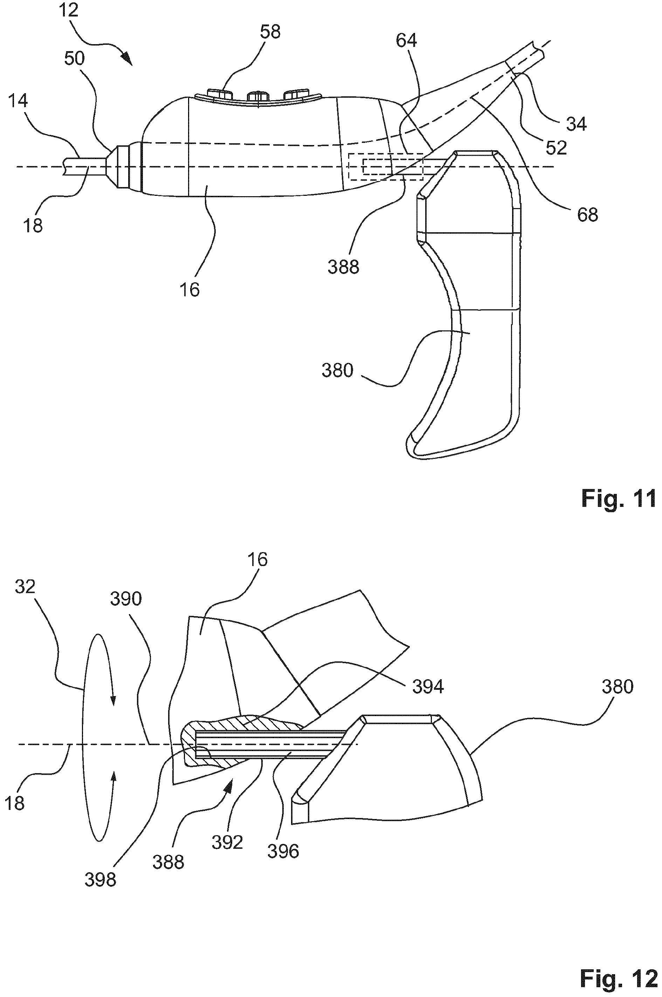

Mit Bezugnahme auf die Figuren 11 und 12 wird eine beispielhafte Ausgestaltung eines Griffstücks 380 veranschaulicht, das in einem Bereich (vergleiche Bezugszeichen 64 in Fig. 3 und Fig. 11) mit dem Handhabungsgehäuse 16 des Instruments 12 koppelbar ist, der näher beim zweiten Ende 52 als beim ersten Ende 50 liegt. Das Griffstück 380 nutzt einen aufgrund der gekrümmten Gestaltung des Handhabungsgehäuses 16 (vergleiche Bezugszeichen 68 in Fig. 11) freien Raum im Bereich der Längsachse 18 benachbart zum proximalen Ende 52 des Handhabungsgehäuses 16.

Die vergrößerte, teilweise geschnittene Darstellung gemäß Fig. 12 veranschaulicht, dass die Schwenkführung 388 konzentrisch zur Längsachse 18 durch den Schaft 14 orientiert ist. Im Ergebnis erfolgt die Schwenkbewegung 32 um eine Schwenkachse 390, die deckungsgleich zur Längsachse 18 ist. Die Schwenkführung 388 umfasst ein männliches Führungsteil 392 und ein weibliches Führungsteil 394, die jeweils einen kreisförmigen Querschnitt aufweisen. Im Ausführungsbeispiel ist das männliches Führungsteil 392 als Führungszapfen 396 ausgebildet, der sich ausgehend vom Griffstück 380 konzentrisch zur Längsachse 18 nach distal erstreckt. Im Ausführungsbeispiel ist das weibliche Führungsteil 394 als Führungsöffnung 398 im Handhabungsgehäuse 16 (vergleiche Bezugszeichen 64 in Fig. 11 zur Veranschaulichung des Bereichs für die Aufnahme) ausgebildet, die konzentrisch zur Längsachse 18 nach proximal geöffnet ist. Mit anderen Worten kann der Führungszapfen 396 entlang der Längsachse 18 in die Führungsöffnung 398 eingeführt werden, um die Schwenkführung 388 auszubilden.

Das Griffstück 380 kann analog zu den vorstehend beschriebenen Ausführungsbeispielen kraftschlüssig und/oder formschlüssig mit dem Handhabungsgehäuse 16 des Instruments 12 gefügt werden. Bei der in den Figuren 11 und 12 gezeigten Gestaltung mit dem Griffstück 380 erlaubt die Schwenkführung 388 eine Übereinstimmung zwischen der Schwenkachse 390 und der Längsachse 18, auch wenn die Haupterstreckungsrichtung 68 (vergleiche Fig. 11) des Handhabungsgehäuses 16 überwiegend deutlich von der Längsachse 18 beabstandet ist. Ganz im Gegenteil, der durch die gekrümmte Gestaltung des Handhabungsgehäuses 16 bereitgestellte Raum um die Längsachse 18 erlaubt die Anordnung und Ausbildung der konzentrischen Schwenkführung 388.

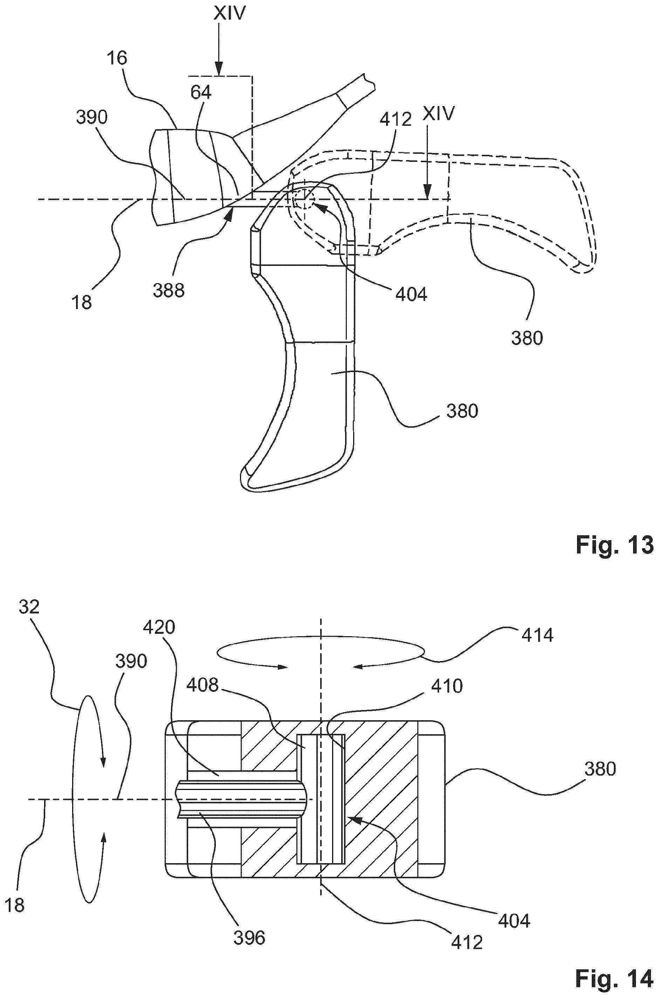

Mit Bezugnahme auf die Figuren 13 und 14 wird eine weitere beispielhafte Ausgestaltung veranschaulicht, die bei der Kopplung zwischen dem Griffstück 380 und dem Handhabungsgehäuse 16 einen weiteren Freiheitsgrad bereitstellt. Zur Vermeidung von Wiederholungen wird auf die zuvor veranschaulichten Ausführungsbeispiele gemäß den Figuren 2-10 und insbesondere auf das anhand der Figuren 11 und 12 veranschaulichte Ausführungsbeispiel verwiesen.

In grundsätzlich zuvor schon beschriebener Weise ist das Griffstück 380 unter Ausbildung einer Schwenkführung 388 um eine Schwenkachse 390 mit dem Handhabungsgehäuse 16 gekoppelt. Fig. 14 zeigt eine schematische vergrößerte Ansicht eines Schnitts durch die Anordnung gemäß Fig. 13 entlang der Linie XIV-XIV. Den Figuren 13 und 14 ist entnehmbar, dass das Griffstück 380 ferner eine Kippführung 404 aufweist, die im Ausführungsbeispiel durch einen in einer Führungsaufnahme 410 gelagerten Bolzen 408 definiert ist. Die Führungsöffnung 410 und der Bolzen 408 definieren eine Kippachse 412, um die eine Kippbewegung 414 ermöglicht ist.

Auf diese Weise kann das Griffstück 380 in Bezug auf die Längsachse 18 bzw. die Schwenkachse 390 beispielhaft zwischen einer senkrechten Orientierung (durchgezogene Linie der Schwenkführung 380 in Fig. 13) und einer parallelen Orientierung (gestrichelte Linie der Schwenkführung 380 in Fig. 13) verschwenkt werden. Andere Kipppositionen und Zwischenpositionen sind denkbar. Auf diese Weise lassen sich weitere Handhabungsposition für das Instrument 12 bereitstellen, die zumindest für bestimmte Anwendungen vorteilhaft sind. In Fig. 14 ist ferner mit 420 eine Freisparung beim Griffstück 380 angedeutet, die eine Bewegung des Führungszapfens 396 relativ zum Griffstück 380 erlaubt, wenn das Griffstück 380 um die Kippachse 412 bewegt wird.

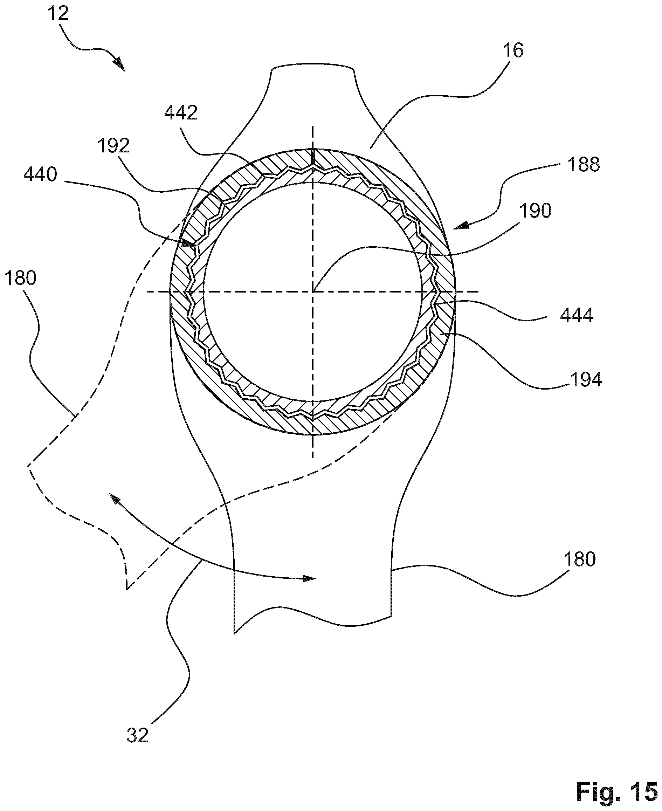

Mit Bezugnahme auf Fig. 15 wird eine weitere beispielhafte Ausgestaltung einer Kopplung zwischen einem Griffstück 180 und einem Handhabungsgehäuse 16 eines Instruments 12 veranschaulicht. Die Schnittebene von Fig. 15 folgt beispielhaft der Linie XV-XV in Fig. 8.

Die Kopplung erfolgt unter Ausbildung einer Schwenkführung 188, die eine Schwenkbewegung 32 um eine Schwenkachse 190 ermöglicht. Die Ansichtsebene in Fig. 15 ist senkrecht zur Schwenkachse 190 (und daher senkrecht zur Längsachse 18, vergleiche hierzu auch die Figuren 2-13).

Die Schwenkführung 188 umfasst in zuvor schon beschriebener Weise ein männliches Führungsteil 192 und ein weibliches Führungsteil 194. Zur Indexierung der Schwenkbewegung 32 ist ein Drehraster 440 vorgesehen, das eine beispielhaft als Außenverzahnung 442 gestaltete Teilung beim männlichen Führungsteil 192 und eine beispielhaft als Innenverzahnung 444 gestaltete Teilung beim weiblichen Führungsteil 194 umfasst. Auf diese Weise kann eine Indexierung mit einem definierten Inkrement für die Schwenkbewegung 32 bereitgestellt werden. Das Drehraster 440 erlaubt eine haptische und/oder akustische Rückmeldung bei der Schwenkbewegung 32.

Die Integration eines Drehrasters 440 kann optional bei den zuovr beschriebenen Schwenkführungen 88, 188, 288, 388 erfolgen, es handelt sich aber nicht notwendigerweise um ein obligatorisches Merkmal. In Fig. 15 sind aus Veranschaulichungsgründen zwei Stellungen (durchgezogene Linien und gestrichelte Linien) des Griffstücks 180 gezeigt. Es versteht sich jedoch, dass üblicherweise das Instrument 12 mit dem Handhabungsgehäuse 16 aktiv relativ zum Griffstück 180 bewegt wird und nicht das Griffstück 180 gegenüber dem Handhabungsgehäuse 16.

## Patentansprüche

1. Chirurgisches Instrument (12), insbesondere endoskopisches Beobachtungsinstrument, mit einem Schaft (14), der sich zwischen einem proximalen Ende (42) und einem distalen Ende (40) erstreckt und eine Längsachse (18) definiert, mit einem Handhabungsgehäuse (16), das an seinem dem Schaft (14) zugewandten ersten Ende (50) an das proximale Ende (42) des Schafts (14) ankoppelt und an seinem vom Schaft (14) abgewandten zweiten Ende (52) einen Leitungsanschluss (34) aufweist, und mit einem Zusatzgriff, wobei das Handhabungsgehäuse (16) zwischen dem ersten Ende (50) und dem zweiten Ende (52) zumindest abschnittsweise gekrümmt ist, so dass der Leitungsanschluss (34) eine gegenüber der Längsachse (18) geneigte Ausrichtung aufweist.

2. Instrument (12) nach Anspruch 1, wobei eine Neigung im Bereich des Leitungsanschlusses (34) 15° bis 45° beträgt, wobei ein Neigungswinkel insbesondere nach proximal geöffnet ist.

3. Instrument (12) nach einem der vorhergehenden Ansprüche, wobei der Leitungsanschluss (34) am von dem Schaft (14) abgewandten Ende des Handhabungsgehäuses (16) von der Längsachse (18) beabstandet ist.

4. Instrument (12) nach einem der vorhergehenden Ansprüche, wobei eine Blickrichtung (46) des Instruments (12) und eine gekrümmte Haupterstreckungsrichtung (68) des Handhabungsgehäuses (16) bezüglich der Längsachse (18) voneinander abgewandt orientiert sind.

5. Instrument (12) nach einem der vorhergehenden Ansprüche, wobei das Handhabungsgehäuse (16) eine Betätigungseinheit zur Steuerung des Instruments (12) aufweist, welche bezüglich einer gekrümmten Haupterstreckungsrichtung (68) des Handhabungsgehäuses (16) zumindest teilweise auf einer Innenseite des Handhabungsgehäuses (16) angeordnet ist.

6. Instrument (12) nach einem der vorhergehenden Ansprüche, wobei das Handhabungsgehäuse (16) und der Schaft (14) drehfest miteinander verbunden sind.

7. Instrument (12) nach einem der vorhergehenden Ansprüche, mit einer beim Handhabungsgehäuse (16) ausgebildeten Aufnahme (60, 62, 64) für den Zusatzgriff, welche eine Anordnungsposition für den Zusatzgriff definiert, die bezüglich einer gekrümmten Haupterstreckungsrichtung (68) des Handhabungsgehäuses (16) auf einer zu einer Innenseite des Handhabungsgehäuses (16) abgewandten Seite des Handhabungsgehäuses (16) angeordnet ist.

8. Instrument (12) nach einem der vorhergehenden Ansprüche, wobei der Zusatzgriff und das Handhabungsgehäuse (16) im montierten Zustand des Zusatzgriffs relativ zueinander um eine Schwenkachse (90, 190, 290, 390) verschwenkbar sind, die im Wesentlichen parallel zur Längsachse (18) des Schafts (14) orientiert ist.

9. Instrument (12) nach einem der vorhergehenden Ansprüche, mit einem Bildaufnehmer (44), der am distalen Ende (40) des Schafts (14) angeordnet ist.

10. Instrument (12) nach einem der vorhergehenden Ansprüche, ferner aufweisend einen Lagesensor (72), der insbesondere als Beschleunigungssensor gestaltet ist, wobei der Lagesensor (72) dazu ausgebildet ist, eine Drehorientierung des Instruments (12) zu erfassen, auf deren Basis eine elektronische Bildaufrichtung von mit dem Instrument (12) erfassten Aufnahmebildern ermöglicht ist.

11. Chirurgisches System (10) mit einem Instrument (12) nach einem der vorhergehenden Ansprüche, ferner aufweisend einen dem Handhabungsgehäuse (16) vorgelagerten Handhabungsabschnitt (20) mit zumindest einer Handhabe (24) zur Steuerung eines Behandlungsvorgangs, insbesondere zur Behandlung von Gewebe und/oder Ablagerungen im Körperinneren eines menschlichen oder tierischen Patienten.
